(19) Europäisches Patentamt
European Patent Office
Office européen des brevets

(11) EP 0 829 258 B1

(12) **FASCICULE DE BREVET EUROPEEN**

(45) Date de publication et mention de la délivrance du brevet:
**03.03.1999 Bulletin 1999/09**

(51) Int. Cl.$^6$: **A61K 7/13**, A61K 7/135, A61K 7/48, A61K 7/06

(21) Numéro de dépôt: **97402050.5**

(22) Date de dépôt: **03.09.1997**

(54) **Composition oxydante gélifiée et utilisations pour la teinture, pour la déformation permanente ou pour la décoloration des cheveux**

Gelierte, oxidative Zusammensetzung und Verwendung zum färben, zur dauerhaften Verformung und zum entfärben der Haaren

Oxidative composition in gel form and uses for dyeing, for permanent deformation or for decoloration of hair

(84) Etats contractants désignés:
**DE ES FR GB IT**

(30) Priorité: **17.09.1996 FR 9611318**

(43) Date de publication de la demande:
**18.03.1998 Bulletin 1998/12**

(73) Titulaire: **L'OREAL**
**75008 Paris (FR)**

(72) Inventeur: **Maubru, Mireille**
**78400 Chatou (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:

| | |
|---|---|
| **EP-A- 0 184 785** | **EP-A- 0 308 825** |
| **EP-A- 0 611 566** | **EP-A- 0 642 781** |
| **FR-A- 2 380 774** | **FR-A- 2 698 004** |
| **FR-A- 2 710 263** | **US-A- 4 781 923** |
| **US-A- 4 804 705** | |

- **CHEMICAL ABSTRACTS, vol. 96, no. 18, 3 mai 1982 Columbus, Ohio, US; abstract no. 148974y, page 405; colonne r; XP002032114 & JP 56 166 109 A (KAO SOAP CO.,LTD)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 829 258 B1

Printed by Xerox (UK) Business Services
2.16.7/3.6

## Description

La présente invention a trait à une composition oxydante gélifiée, destinée au traitement des matières kératiniques comprenant un polymère poly(acide 2-acrylamido 2-méthylpropane sulfonique) réticulé et neutralisé à au moins 90% ainsi que ses utilisations pour la teinture, pour la déformation permanente ou pour la décoloration des cheveux.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants. On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène ou des composés susceptibles de produire le peroxyde d'hydrogène par hydrolyse tels que le peroxyde d'urée, les persels comme les perborates et persulfates. Le peroxyde d'hydrogène est particulièrement préféré.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéine et ses divers dérivés, la cystéamine et ses dérivés, l'acide thiolactique ou l'acide thioglycolique, leurs sels ainsi que leurs esters, notamment le thioglycolate de glycérol.

Concernant les compositions oxydantes nécessaires à la mise en oeuvre de l'étape de fixation, on fait le plus souvent appel, dans la pratique, à des compositions à base d'eau oxygénée.

On recherche depuis de nombreuses années des formulations cosmétiques sous forme de gels transparents. Ce type de présentation est très appréciée par le consommateur pour des raisons esthétiques et pour des raisons de facilité et de confort d'utilisation.

La forme gel correspond le plus souvent à une préoccupation pratique pour le formulateur : faciliter la prise du produit hors de son conditionnement sans perte significative, limiter la diffusion du produit à la zone locale de traitement et pouvoir l'utiliser dans des quantités suffisantes pour obtenir l'effet cosmétique recherché. Cet objectif est important pour les formulations oxydantes utilisées en teinture capillaire, pour les permanentes ou pour la décoloration des cheveux. Celles-ci doivent bien s'étaler et se répartir de façon régulière le long des fibres kératiniques et ne pas couler sur le front, la nuque, le visage ou dans les yeux.

Il est généralement difficile d'obtenir des gels à base de peroxyde tel que le peroxyde d'hydrogène stables à la conservation en utilisant des épaississants et/ou des gélifiants hydrosolubles classiques, par exemple ceux du type polymère acrylique réticulé comme ceux vendus sous le nom CARBOPOL par la société GOODRICH. Les peroxydes sont utilisés en cosmétique sous forme de solutions aqueuses acides pour des raisons de stabilité. Ils conduisent le plus souvent en présence des gélifiants classiques à des variations sensibles de la viscosité du gel durant le stockage.

On connaît dans le brevet US 4.804.705, des gels à base de peroxyde d'hydrogène contenant un gélifiant formé par réaction d'une hydroxyéthylcellulose quaternisée du type CELQUAT (produit vendu par NATIONAL STARCH), d'une solution aqueuse à 15% en poids d'un polymère poly(acide 2-acrylamido 2-méthylpropane sulfonique) non-réticulé du type COSMEDIA HSP-1180 (produit vendu par HENKEL) et d'un polystyrène sulfonate de sodium du type FLEXAN 3 (produit vendu par NATIONAL STARCH), utilisés dans des concentrations particulières.

La demanderesse a découvert de manière surprenante une nouvelle famille d'agents épaississants et/ou gélifiants permettant d'obtenir des gels transparents à base de peroxyde d'hydrogène ou de composé oxydant susceptible de libérer du peroxyde d'hydrogène, stables au stockage. Il s'agit de polymères poly(acide 2-acrylamido 2-méthylpropane sulfonique) réticulés et neutralisés à au moins 90%. Ces nouveaux agents gélifiants n'affectent pas les propriété oxydantes du peroxyde d'hydrogène ou d'un composé susceptible de produire le peroxyde d'hydrogène par hydrolyse présent dans le gel ainsi formé.

La présente invention a donc pour objet une composition cosmétique et/ou dermatologique destinée au traitement des matières kératiniques comprenant dans un support approprié pour les matières kératiniques :

(a) au moins un polymère poly(acide 2-acrylamido 2-méthylpropane sulfonique) réticulé et neutralisé à au moins 90% et

(b) au moins un oxydant choisi dans le groupe constitué par le peroxyde d'hydrogène et les composés susceptibles de produire par hydrolyse du peroxyde d'hydrogène ou leurs mélanges.

L'invention concerne également l'utilisation de ces polymères comme agent épaississant et/ou gélifiant dans des compositions cosmétiques et/ou dermatologiques comprenant au moins un oxydant choisi dans le groupe constitué par le peroxyde d'hydrogène et les composés susceptibles de produire par hydrolyse du peroxyde d'hydrogène ou leurs mélanges.

Les polymères poly(acide 2-acrylamido 2-méthylpropane sulfonique) réticulés et pratiquement ou totalement neutralisés, conformes à l'invention sont en général caractérisés par le fait qu'ils comprennent, distribués de façon aléatoire :

a) de 90 à 99,9% en poids de motifs de formule générale (1) suivante :

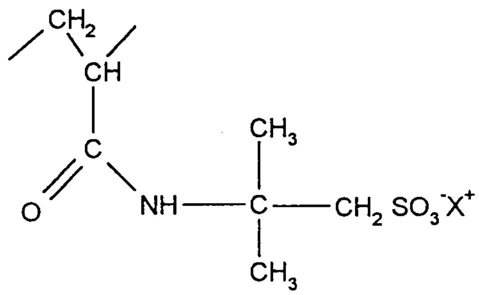

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

De façon préférentielle, les polymères de l'invention comportent un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

Les polymères selon l'invention plus particulièrement préférés comprennent de 98 à 99,5 % en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Les monomères de réticulation ayant au moins deux doubles-liaisons oléfiniques sont choisis par exemple parmi le dipropylèneglycol-diallyléther, les polyglycoldiallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropanediallyléther, le méthylen-bis-acrylamide ou le divinylbenzène.

Les monomères de réticulation ayant au moins deux doubles-liaisons oléfiniques sont plus particulièrement choisis parmi ceux répondant à la formule générale (2) suivante :

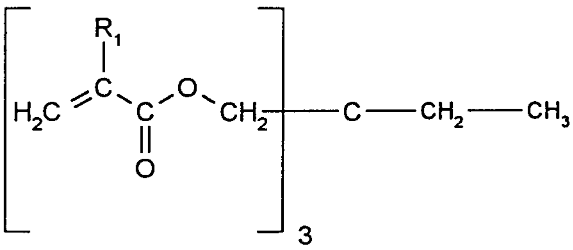

(2)

dans laquelle $R_1$ désigne un atome d'hydrogène ou un alkyle en $C_1$-$C_4$ et plus particulièrement méthyle (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'electrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec:

- M désignant la masse en grammes de la macromolécule non-dissoute ;
- $N_A$ désignant le nombre d'Avogadro ;
- $V_1$ désignant le volume spécifique du solvant;
- $V_2$ désignant le volume spécifique de la macromolécule ;
- d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoides à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution , on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi_\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères

par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W.; Comp. Phys., 27, 213, 1982 ;
(5) Provincher S.W.; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al , Macromolecules, 1995, 28,4914-4919.

Les polymères particulièrement préférés sont ceux présentant une viscosité mesurée au viscosimètre BROOKFIELD, mobile 4, à une vitesse de rotation de 100 tours/minutes dans une solution d'eau à 2 % et à 25 °C supérieure ou égale à 1000 cps et plus préférentiellement allant de 5000 à 40000 cps et plus particulièrement de 6500 à 35000 cps.

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol ;

(b) on neutralise la solution ou la dispersion de monomère obtenue en (a) par une ou plusieurs bases minérales ou organiques, de préférence l'ammoniaque $NH_3$ , dans une quantité permettant d'obtenir un taux de neutralisation des fonctions acides sulfoniques du polymère allant de 90 à 100% ;

(c) on ajoute à la solution ou dispersion obtenue en (b), le ou les monomères réticulants ;

(d) on effectue une polymérisation radicalaire classique en la présence d'amorceurs de radicaux libres à une température allant de 10 à 150°C ; le polymère précipitant dans la solution ou la dispersion à base de tertio-butanol.

Les poly(acide 2-acrylamido 2-méthylpropane sulfonique) réticulés, pratiquement ou totalement neutralisés sont présents dans les compositions cosmétiques ou dermatologiques de l'invention dans des concentrations allant préférentiellement de 0,01 à 10% en poids par rapport au poids total de la composition et plus préférentiellement de 0,05 à 5% en poids.

L'agent oxydant de la composition selon l'invention est choisi de préférence dans le groupe formé par l'eau oxygénée, le peroxyde d'urée, les persels tels que les perborates ou les persulfates ou leurs mélanges.

De préférence, l'agent oxydant est le peroxyde d'hydrogène.

La concentration en peroxyde d'hydrogène peut varier de 0,5 à 40 volumes, de préférence de 2 à 30 volumes et celle en composé susceptible de former du peroxyde d'hydrogène par hydrolyse de 0,1 à 25 % en poids par rapport au poids total de la composition oxydante.

Les compositions oxydantes selon l'invention peuvent être anhydres ou aqueuses.

Les compositions oxydantes selon l'invention sont de préférence aqueuses et le pH de l'ensemble d'une composition oxydante aqueuse varie de préférence de 1 à 13, et encore plus préférentiellement de 2 à 12.

La composition oxydante peut également se présenter, en particulier dans le cas de la décoloration, sous forme de deux parties à mélanger au moment de l'emploi, l'une de ces deux parties contenant des agents alcalins et se présentant sous forme solide ou liquide. Pour le peroxyde d'hydrogène , le pH est de préférence inférieur à 7 avant mélange.

Le pH des compositions aqueuses oxydantes selon l'invention peut être obtenu et/ou ajusté classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

La composition oxydante peut contenir des additifs connus pour leur utilisation dans les compositions oxydantes pour la teinture des cheveux par oxydation, pour la déformation permanente ou la décoloration des cheveux tels que des agents alcalinisants ou acidifiants, des agents conservateurs, des agents séquestrants, des opacifiants et éventuellement un agent de conditionnement. Elle peut se présenter sous la forme d'un shampooing.

Les compositions selon l'invention se présentent généralement sous la forme d'un gel transparent. Leur viscosité varie de préférence de 50 centipoises à 100 poises et plus préférentiellement de 75 centipoises à 5 poises.

Un autre objet de l'invention est un procédé de teinture d'oxydation des fibres kératiniques et en particulier des fibres kératiniques humaines telles que les cheveux mettant en oeuvre une composition tinctoriale comprenant dans un support approprié pour la teinture des fibres kératiniques au moins un précurseur de colorant d'oxydation et une composition oxydante telle que définie ci-dessus.

Selon ce procédé, on applique sur les fibres au moins une composition tinctoriale telle que définie précédemment, la couleur étant révélée à pH acide, neutre ou alcalin à l'aide d'une composition oxydante selon l'invention qui est appliquée simultanément ou séquentiellement, avec ou sans rinçage intermédiaire.

Selon une forme de mise en oeuvre particulièrement préférée du procédé de teinture selon l'invention, on mélange, au moment de l'emploi, la composition tinctoriale décrite ci-dessus avec une composition oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

Un autre objet de la présente invention est un procédé de déformation permanente des fibres kératiniques, et en particulier des cheveux, utilisant comme composition oxydante la composition définie ci-dessus.

La première étape de ce procédé consiste à appliquer sur les cheveux une composition réductrice. Cette application se fait mèche par mèche ou globalement.

La composition réductrice comprend au moins un agent réducteur, qui peut être en particulier choisi parmi l'acide thioglycolique, la cystéine, la cystéamine, le thioglycolate de glycérol, l'acide thiolactique, ou les sels des acides thiolactique ou thioglycolique.

L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Les cheveux peuvent également être mis en forme sans l'aide de moyens extérieurs, simplement avec les doigts.

Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.

Dans la deuxième étape, facultative, du procédé (étape (ii)), les cheveux imprégnés de la composition réductrice sont donc ensuite rincés soigneusement par une composition aqueuse.

Puis, dans une troisième étape (étape (iii)), on applique sur les cheveux ainsi rincés la composition oxydante de l'invention, dans le but de fixer la nouvelle forme imposée aux cheveux.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées selon l'invention est de préférence l'eau ou une solution hydroalcoolique d'un alcool inférieur tel que l'éthanol, l'isopropanol ou le butanol.

L'eau oxygénée peut être stabilisée par exemple par la phénacétine, l'acétanilide, les phosphates mono et trisodiques ou par le sulfate d'hydroxy-8 quinoléine, les stannates dont le stannate de sodium.

Si la tension des cheveux était maintenue par des moyens extérieurs, on peut retirer de la chevelure ces derniers (rouleaux, bigoudis et analogues) avant ou après l'étape de fixation.

Enfin, dans la dernière étape du procédé selon l'invention (étape (iv)), étape facultative également, les cheveux imprégnés de la composition oxydante sont rincés soigneusement, généralement à l'eau.

On obtient finalement une chevelure facile à démêler, douce. Les cheveux sont ondulés.

La composition oxydante selon l'invention peut également être utilisée dans un procédé de décoloration des fibres kératiniques, et en particulier des cheveux.

Le procédé de décoloration selon l'invention comprend une étape d'application sur les fibres kératiniques d'une composition oxydante selon l'invention, cette composition comprenant de préférence de l'eau oxygénée en milieu alcalin après mélange extemporané. Classiquement, une deuxième étape du procédé de décoloration selon l'invention est une étape de rinçage des fibres kératiniques.

Des exemples concrets illustrant l'invention vont maintenant être donnés.

Dans ce qui suit ou ce qui précède, sauf mention contraire, les pourcentages sont exprimés en poids.

Les exemples suivants illustrent l'invention sans présenter un caractère limitatif.

***EXEMPLE DE PREPARATION***

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures.On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minutes dans une solution d'eau à 2 % et à 25 °C de l'ordre de 7000 cps.

Le rayon hydrodynamique du polymère obtenu dans une solution aqueuse détérminé par diffusion de la lumière dynamique est de 160 nm.

***EXEMPLE 1:***

La demanderesse a réalisé un test comparatif afin de mettre en évidence l'amélioration apportée au niveau de la conservation après deux mois de stockage d'une composition oxydante.

On a réalisé la composition oxydante $A_1$ conforme à l'invention, suivante :

| | |
|---|---|
| - Poly(acide 2-acrylamido 2-méthylpropane sulfonique) réticulé et neutralisé par de l'ammoniaque préparé selon le procédé de l'exemple de préparation de viscosité de l'ordre de 7.000 cps dans une ..solution d'eau à 2% et à 25°C | 0,27 g |
| - Peroxyde d'hydrogène à 200 volumes | 12 g |
| - Stabilisant | qs |
| - Agent de pH | qs pH 4,7 |
| - Eau déminéralisée | qsp 100 g |

La viscosité instantanée de la composition $A_1$ est de l'ordre de 150 cps.

On a également réalisé une composition oxydante $B_1$, comparative, de même composition que A mais contenant à la place du poly(acide 2-acrylamido 2-méthylpropane sulfonique) réticulé et neutralisé un poly(acide acrylique) réticulé vendu sous le nom CARBOPOL 940 par la société GOODRICH à raison de 0,18% en poids pour obtenir un gel ayant une viscosité de l'ordre de 150 cps.

On a également réalisé une composition oxydante $C_1$, comparative, de même composition que $A_1$ mais contenant à la place du poly(acide 2-acrylamido 2-méthylpropane sulfonique) réticulé et neutralisé, un poly(acide acrylique) réticulé vendu sous le nom CARBOPOL 980 par la société GOODRICH à raison de 0,17% en poids pour obtenir un gel ayant une viscosité de l'ordre de 150 cps.

Chacune des compositions $A_1$, $B_1$, $C_1$ est stockée pendant 2 mois à 45°C. On calcule ensuite pour chaque composition la variation relative de la viscosité pendant la période de stockage.

Les résultats du test sont résumés dans le tableau suivant.

| *Formule* | *Variation de la viscosité après 2 mois de stockage à 45°C* |
|---|---|
| Composition $A_1$ (invention) | +3% |
| Composition $B_1$ (comparatif) | -18% |
| Composition $C_1$ (comparatif) | -23% |

Ces résultats montrent clairement que l'utilisation d'un poly(acide 2-acrylamido 2-méthylpropane sulfonique) réticulé et neutralisé à au moins 90%en poids dans une composition oxydante d'un procédé de déformation permanente améliore sa stabilité au stockage contrairement à un gélifiant classique du type CARBOPOL.

***EXEMPLE 2*: PROCEDE DE COLORATION PAR OXYDATION**

***Composition tinctoriale $A_2$* :**

| | |
|---|---|
| - Paraphénylènediamine | 0,324 g |
| - 1-β hydroxyéthyloxy 2,4-diaminobenzène dichlorhydrate | 0,723 g |
| - Alcool oléique polyglycérolé à 2 moles de glycérol | 4,0 g |
| - Alcool oléique polyglycérolé à 4 moles de glycérol à 78 % de matières actives (M.A.) | 5,69 g M.A. |
| - Acide oléique | 3,0 g |
| - Amine oléique à 2 moles d'oxyde d'éthylène vendue sous la dénomination commerciale ETHOMEEN O12 par la société AKZO | 7,0 g |
| - Laurylamino succinamate de diéthylaminopropyle, sel de sodium à 55 % de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium à en solution aqueuse à 35 % de M.A. | 0,455 g M.A. |
| - Acétate d'ammonium | 0,8 g |
| - Antioxydant, séquestrant | q.s. |
| - Parfum, conservateur | q.s. |
| - Ammoniaque à 20 % de $NH_3$ | 10,0 g |
| - Eau déminéralisée | qsp 100 g |

***Composition oxydante $B_2$:***

| | |
|---|---|
| - Poly(acide 2-acrylamido 2-méthylpropane sulfonique) réticulé et neutralisé par de l'ammoniaque préparé selon le procédé de l'exemple de préparation de viscosité de l'ordre de 7.000 cps dans une ..solution d'eau à 2% et à 25°C | 0,27 g |
| - Peroxyde d'hydrogène à 200 volumes | 12 g |
| - Stabilisant | qs |
| - Acide orthophosphorique | qs pH 3,8 |
| - Eau déminéralisée | qsp 100 g |

Au moment de l'emploi, la composition $A_2$ a été mélangée à la composition oxydante $B_2$.

Le mélange obtenu a été appliqué pendant 30 minutes, sur des mèches de cheveux gris naturels à 90 % de blancs, permanentés ou non, à raison de 28 g pour 3 g.

On obtient des colorations bleues puissantes.

***EXEMPLE 3: PROCEDE DE DEFORMATION PERMANENTE***

Un exemple concret de composition réductrice $A_3$ et de composition fixatrice $B_3$ (oxydante) pour un procédé de déformation permanente des cheveux est donné ci-après :

***Réducteur $A_3$:***

| | |
|---|---|
| - Cocoylbétaïne | 2,0 % |
| - Acide thioglycolique | 7,0 % |
| - Bicarbonate d'ammonium | 5,5 % |
| - Séquestrant | 0,4 % |
| - Ammoniaque à 20 % $NH_3$ | 6,6 % |
| - Eau déminéralisée | qsp 100 % |

La composition réductrice a été réalisée par simple mélange.

***Fixateur $B_3$:***

| | |
|---|---|
| - Poly(acide 2-acrylamido 2-méthylpropane sulfonique) réticulé et neutralisé par de l'ammoniaque préparé selon le procédé de l'exemple de préparation de viscosité de l'ordre de 7.000 cps dans une ..solution d'eau à 2% et à 25°C | 0,3 g |
| - Peroxyde d'hydrogène à 200 volumes | 4,8 g |
| - Stabilisant | qs |
| - Acide citrique | qs pH 4 |
| - Eau déminéralisée | qsp 100 g |

## Revendications

1. Composition cosmétique et/ou dermatologique destinée au traitement des matières kératiniques, caractérisée par le fait qu'elle comprend dans un support approprié pour les matières kératiniques :

   (a) au moins un polymère poly(acide 2-acrylamido 2-méthylpropane sulfonique) réticulé et neutralisé à au moins 90% et
   (b) au moins un oxydant choisi dans le groupe constitué par le peroxyde d'hydrogène et les composés susceptibles de produire par hydrolyse du peroxyde d'hydrogène ou leurs mélanges.

2. Composition selon la revendication 1, caractérisée par le fait que le poly(acide 2-acrylamido 2-méthylpropane sulfonique) réticulé comprend, distribués de façon aléatoire :

   a) de 90 à 99,9% en poids de motifs de formule générale (1) suivante :

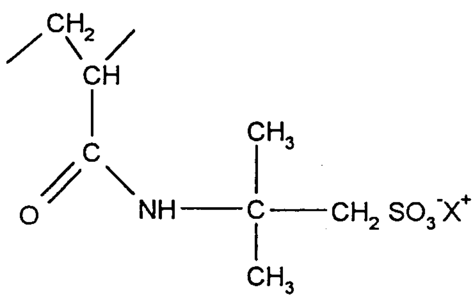 (1)

   dans laquelle $X^+$ désigne un cation ou un mélange de cations, au plus 10% mol des cations $X^+$ pouvant être des protons $H^+$ ;
   b) de 0,01 à 10% en poids de motifs réticulants provenant d'au moins un monomère ayant au moins deux doubles-liaisons oléfiniques ; les proportions en poids étant définis par rapport au poids total du polymère.

3. Composition selon la revendication 2, caractérisée par le fait que le poly(acide 2-acrylamido 2-méthylpropane sulfonique) réticulé comporte un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

4. Composition selon la revendication 2 ou 3, caractérisée par le fait que le poly(acide 2-acrylamido 2-méthylpropane sulfonique) réticulé comporte de 98 à 99,5 % en poids de motifs de formule (1) et de 0,2 à 2 % en poids de motifs réticulants.

5. Composition selon l'une quelconque des revendications 1 à 4, caractérisée par le fait que dans la formule (1) le cation $X^+$ est $NH_4^+$.

6. Composition selon l'une quelconque des revendications 1 à 5, caractérisée par le fait que les monomères réticulants répondent à la formule générale (2) suivante:

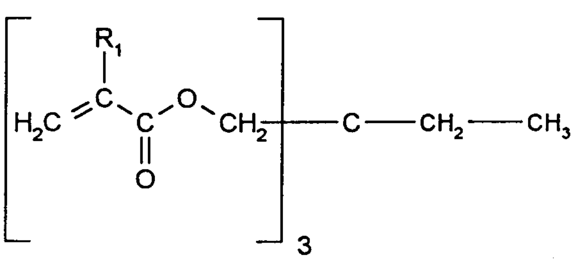 (2)

   dans laquelle $R_1$ désigne hydrogène ou un alkyle en $C_1$-$C_4$.

7. Composition selon l'une quelconque des revendications 1 à 6, caractérisée par le fait que le poly(acide 2-acrylamido 2-méthylpropane sulfonique) est réticulé par le triméthylol propane triacrylate.

8. Composition selon l'une quelconque des revendications 1 à 7, caractérisée par le fait que le poly(acide 2-acrylamido 2-méthylpropane sulfonique) réticulé présente une viscosité mesurée au viscosimètre BROOKFIELD dans une solution d'eau à 2 % et à 25 °C supérieure ou égale à 1000 cps.

9. Composition selon l'une quelconque des revendications 1 à 8, caractérisée par le fait que le poly(acide 2-acrylamido 2-méthylpropane sulfonique) réticulé présente une viscosité mesurée au viscosimètre BROOKFIELD dans une solution d'eau à 2 % et à 25 °C allant de 5000 à 40000 cps et plus particulièrement de 6500 à 35000 cps.

10. Composition selon l'une quelconque des revendications 1 à 9, caractérisée par le fait que le poly(acide 2-acrylamido 2-méthylpropane sulfonique) réticulé est présent dans dans des concentrations allant préférentiellement de 0,01 à 10% en poids par rapport au poids total de la composition et plus préférentiellement de 0,05 à 5% en poids.

11. Composition selon l'une quelconque des revendications 1 à 10, caractérisée par le fait que l'agent oxydant est choisi dans le groupe formé par le peroxyde d'hydrogène, le peroxyde d'urée, les perborates, les persulfates ou leurs mélanges.

12. Composition selon l'une quelconque des revendications 1 à 11, caractérisée par le fait que l'agent oxydant est le peroxyde d'hydrogène.

13. Composition selon la revendication 12, caractérisée par le fait que la concentration en peroxyde d'hydrogène varie de 0,5 à 40 volumes et de préférence de 2 à 30 volumes

14. Composition selon la revendication 11, caractérisée par le fait que la concentration en composé susceptible de produire par hydrolyse du peroxyde d'hydrogène varie de 0,1 à 25 % en poids par rapport au poids total de la composition oxydante.

15. Composition selon l'une quelconque des revendications 1 à 14, caractérisée par le fait qu'elle est aqueuse et que le pH de l'ensemble de la composition varie de 1 à 13, et encore plus préférentiellement de 2 à 12.

16. Composition selon l'une quelconque des revendications 1 à 15, caractérisée par le fait qu'elle se présente sous la forme d'un gel transparent de viscosité allant de 50 centipoises à 100 poises et plus préférentiellement de 75 centipoises à 5 poises.

17. Procédé de teinture d'oxydation des fibres kératiniques et en particulier des fibres kératiniques humaines telles que les cheveux mettant en oeuvre une composition tinctoriale comprenant dans un support approprié pour la teinture des fibres kératiniques au moins un précurseur de colorant d'oxydation et une composition oxydante telle que définie dans l'une quelconque des revendications 1 à 16.

18. Procédé de teinture selon la revendication 17 selon lequel on mélange, au moment de l'emploi, la composition tinctoriale avec la composition oxydante ; le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

19. Procédé de teinture selon la revendication 17 selon lequel on applique séquentiellement la composition tinctoriale et la composition oxydante, avec ou sans rinçage intermédiaire.

20. Procédé de traitement des matières kératiniques, en particulier des cheveux, en vue d'obtenir une déformation permanente de ces dernières, en particulier sous la forme de cheveux permanentés, ce procédé comprenant les étapes suivantes : (i) on applique sur la matière kératinique à traiter une composition réductrice, la matière kératinique étant mise sous tension mécanique avant, pendant, ou après ladite application, (ii) on rince éventuellement la matière kératinique, (iii) on applique sur la matière kératinique éventuellement rincée une composition oxydante telle que définie à l'une des revendications 1 à 16, (iv) on rince éventuellement à nouveau la matière kératinique.

21. Procédé de décoloration des matières kératiniques, en particulier des cheveux, comprenant les étapes suivantes : i) on applique sur la matière kératinique une composition oxydante selon l'une quelconque des revendications 1 à

16, ii) on rince la matière kératinique ainsi traitée.

**22.** Utilisation comme agent épaississant et/ou gélifiant dans une composition cosmétique et/ou dermatologique comprenant au moins un oxydant choisi dans le groupe constitué par le peroxyde d'hydrogène et les composés susceptibles de produire par hydrolyse du peroxyde d'hydrogène ou leurs mélanges, d'un polymère poly(acide 2-acrylamido 2-méthylpropane sulfonique) réticulé et neutralisé tel que défini dans les revendications 1 à 10.

## Claims

**1.** Cosmetic and/or dermatological composition intended for treating keratin substances, characterized in that it comprises, in a support suitable for keratin substances:

(a) at least one crosslinked and at least 90 % neutralized poly(2-acrylamido-2-methylpropanesulphonic acid) polymer and
(b) at least one oxidizing agent chosen from the group consisting of hydrogen peroxide and compounds capable of producing hydrogen peroxide by hydrolysis, or mixtures thereof.

**2.** Composition according to Claim 1, characterized in that the crosslinked poly(2-acrylamido-2-methylpropanesulphonic acid) comprises, distributed randomly:

a) from 90 to 99.9 % by weight of units of general formula (1) below:

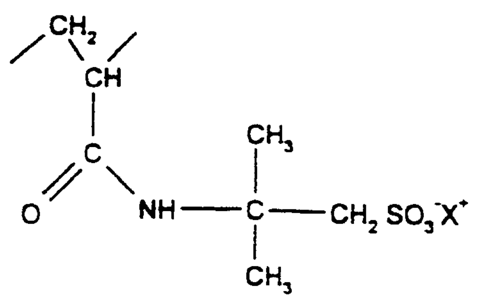

(1)

in which $X^+$ denotes a cation or a mixture of cations, not more than 10 mol % of which cations $X^+$ may be protons $H^+$;
b) from 0.01 to 10 % by weight of crosslinking units derived from at least one monomer having at least two olefinic double bonds; the weight proportions being defined relative to the total weight of the polymer.

**3.** Composition according to Claim 2, characterized in that the crosslinked poly(2-acrylamido-2-methylpropanesulphonic acid) contains a number of units of formula (1) in a sufficiently large amount to obtain polymer particles whose hydrodynamic volume in aqueous solution has a radius ranging from 10 to 500 nm and whose distribution is homogeneous and unimodal.

**4.** Composition according to Claim 2 or 3, characterized in that the crosslinked poly(2-acrylamido-2-methylpropanesulphonic acid) contains from 98 to 99.5 % by weight of units of formula (1) and from 0.2 to 2 % by weight of crosslinking units.

**5.** Composition according to any one of Claims 1 to 4, characterized in that, in formula (1), the cation $X^+$ is $NH_4^+$.

**6.** Composition according to any one of Claims 1 to 5, characterized in that the crosslinking monomers correspond to general formula (2) below:

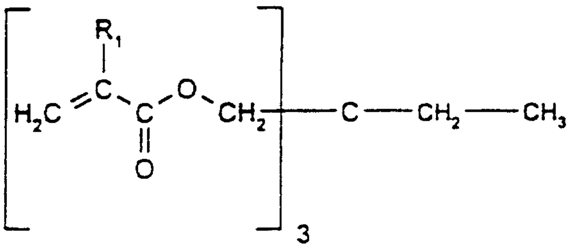

(2)

in which $R_1$ denotes hydrogen or a $C_1$-$C_4$ alkyl.

**7.** Composition according to any one of Claims 1 to 6, characterized in that the poly(2-acrylamido-2-methylpropanesulphonic acid) is crosslinked with trimethylolpropane triacrylate.

**8.** Composition according to any one of Claims 1 to 7, characterized in that the crosslinked poly(2-acrylamido-2-methylpropanesulphonic acid) has a viscosity, measured with a Brookfield viscometer in an aqueous 2 % solution and at 25°C, of greater than or equal to 1000 cps.

**9.** Composition according to any one of Claims 1 to 8, characterized in that the crosslinked poly(2-acrylamido-2-methylpropanesulphonic acid) has a viscosity, measured with a Brookfield viscometer in an aqueous 2 % solution and at 25°C, ranging from 5000 to 40,000 cps and more particularly from 6500 to 35,000 cps.

**10.** Composition according to any one of Claims 1 to 9, characterized in that the crosslinked poly(2-acrylamido-2-methylpropanesulphonic acid) is present in concentrations preferably ranging from 0.01 to 10 % by weight relative to the total weight of the composition and more preferably from 0.05 to 5 % by weight.

**11.** Composition according to any one of Claims 1 to 10, characterized in that the oxidizing agent is chosen from the group formed by hydrogen peroxide, urea peroxide, perborates and persulphates, or mixtures thereof.

**12.** Composition according to any one of Claims 1 to 11, characterized in that the oxidizing agent is hydrogen peroxide.

**13.** Composition according to Claim 12, characterized in that the hydrogen peroxide concentration ranges from 0.5 to 40 volumes and preferably from 2 to 30 volumes.

**14.** Composition according to Claim 11, characterized in that the concentration of compound capable of producing hydrogen peroxide by hydrolysis ranges from 0.1 to 25 % by weight relative to the total weight of the oxidizing composition.

**15.** Composition according to any one of Claims 1 to 14, characterized in that it is aqueous and in that the pH of the entire composition ranges from 1 to 13 and even more preferably from 2 to 12.

**16.** Composition according to any one of Claims 1 to 15, characterized in that it is in the form of a transparent gel with a viscosity ranging from 50 centipoises to 100 poises and more preferably from 75 centipoises to 5 poises.

**17.** Process for the oxidation dyeing of keratin fibres, and in particular human keratin fibres such as the hair, using a dye composition comprising, in a support which is suitable for dyeing keratin fibres, at least one oxidation dye precursor and one oxidizing composition as defined in any one of Claims 1 to 16.

**18.** Dyeing process according to Claim 17, according to which the dye composition is mixed, at the time of use, with the oxidizing composition; the mixture obtained is then applied to the keratin fibres and is left in place for 3 to 50 minutes approximately, preferably 5 to 30 minutes approximately, after which the fibres are rinsed, washed with shampoo, rinsed again and dried.

**19.** Dyeing process according to Claim 17, according to which the dye composition and the oxidizing composition are applied sequentially, with or without intermediate rinsing.

20. Process for treating keratin substances, in particular hair, in order to obtain a permanent reshaping of the hair, in particular in the shape of permanent-waved hair, this process comprising the following steps: (i) a reducing composition is applied to the keratin substance to be treated, the keratin substance being placed under mechanical tension before, during or after the said application, (ii) the keratin substance is optionally rinsed, (iii) an oxidizing composition as defined in any one of Claims 1 to 16 is applied to the optionally rinsed keratin substance, and (iv) the keratin substance is optionally rinsed again.

21. Process for bleaching keratin substances, in particular hair, comprising the following steps: i) an oxidizing composition according to any one of Claims 1 to 16 is applied to the keratin substance, and (ii) the keratin substance thus treated is rinsed.

22. Use as a thickener and/or gelling agent in a cosmetic and/or dermatological composition comprising at least one oxidizing agent chosen from the group consisting of hydrogen peroxide and compounds capable of producing hydrogen peroxide by hydrolysis, or mixtures thereof, of a crosslinked and neutralized poly(2-acrylamido-2-methylpropanesulphonic acid) polymer as defined in Claims 1 to 10.

**Patentansprüche**

1. Kosmetische und/oder dermatologische Zusammensetzungen, die zur Behandlung von Keratinmaterialien bestimmt sind, dadurch gekennzeichnet, daß sie in einem für Keratinmaterialien geeigneten Träger folgende Bestandteile enthalten:

   a) mindestens ein Poly(2-acrylamido-2-methylpropan-sulfonsäure)-Polymer, das vernetzt und zu mindestens 90% neutralisiert ist, und
   b) mindestens ein Oxidationsmittel, das unter Wasserstoffperoxid und den Verbindungen, die befähigt sind, durch Hydrolyse Wasserstoffperoxid freizusetzen, sowie den Gemischen dieser Oxidationsmittel ausgewählt ist.

2. Zusammensetzungen nach Anspruch 1, dadurch gekennzeichnet, daß die vernetzte Poly-(2-acrylamido-2-methylpropan-sulfonsäure) aus folgenden, zufällig verteilten Struktureinheiten besteht:

   a) zu 90 bis 99,9 Gew.-% aus Einheiten der folgenden allgemeinen Formel (1):

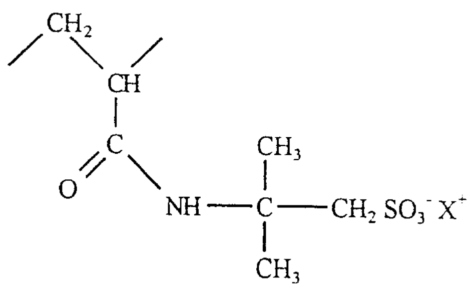

(1),

   in der $X^+$ ein Kation oder ein Gemisch von Kationen bezeichnet, wobei maximal 10 mol-% der Kationen ($X^+$) Protonen ($H^+$) sein können;
   und

   b) zu 0,01 bis 10 Gew.-% aus vernetzenden Einheiten, die aus mindestens einem Monomeren stammen, das mindestens zwei olefinische Doppelbindungen aufweist; hierbei sind die Gewichtsanteile auf das Gesamtgewicht des Polymeren bezogen.

3. Zusammensetzungen nach Anspruch 2, dadurch gekennzeichnet, daß die vernetzte Poly-(2-acrylamido-2-methylpropan-sulfonsäure) eine ausreichend hohe Anzahl von Einheiten der Formel (1) enthält, um Polymerteilchen zu bilden, deren hydrodynamisches Volumen in wässeriger Lösung einem Teilchenradius im Bereich von 10 bis 500 nm entspricht und die eine homogene und unimodale Verteilung aufweisen.

4. Zusammensetzungen nach Anspruch 2 oder 3, dadurch gekennzeichnet, daß die vernetzte Poly-(2-acrylamido-2-methylpropan-sulfonsäure) zu 98 bis 99,5 Gew.-% aus Einheiten der Formel (1) und zu 0,2 bis 2 Gew.-% aus vernetzenden Einheiten besteht.

5. Zusammensetzungen nach einem der Ansprüche 1 bis 4, dadurch gekennzeichnet, daß in Formel (1) das Kation ($X^+$) $NH_4^+$ bezeichnet.

6. Zusammensetzungen nach einem der Ansprüche 1 bis 5, dadurch gekennzeichnet, daß die vernetzenden Monomeren der folgenden allgemeinen Formel (2) entsprechen:

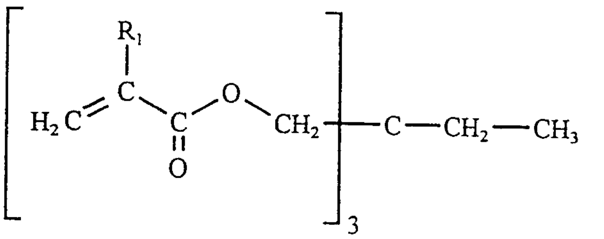 (2),

in der $R_1$ ein Wasserstoffatom oder einen $C_{1-4}$-Alkylrest bezeichnet.

7. Zusammensetzungen nach einem der Ansprüche 1 bis 6, dadurch gekennzeichnet, daß die Poly-(2-acrylamido-2-methylpropan-sulfonsäure) mit Trimethylolpropan-triacrylat vernetzt ist.

8. Zusammensetzungen nach einem der Ansprüche 1 bis 7, dadurch gekennzeichnet, daß die vernetzte Poly-(2-acrylamido-2-methylpropan-sulfonsäure) eine mit Hilfe eines BROOKFIELD-Viskosimeters bei 25 °C in 2%-iger wässeriger Lösung bestimmte Viskosität größer oder gleich 1 000 cP aufweist.

9. Zusammensetzungen nach einem der Ansprüche 1 bis 8, dadurch gekennzeichnet, daß die vernetzte Poly-(2-acrylamido-2-methylpropan-sulfonsäure) eine mit Hilfe eines BROOKFIELD-Viskosimeters bei 25 °C in 2%-iger wässeriger Lösung bestimmte Viskosität im Bereich von 5 000 bis 40 000 cP und insbesondere von 6 500 bis 35 000 cP aufweist.

10. Zusammensetzungen nach einem der Ansprüche 1 bis 9, dadurch gekennzeichnet, daß die vernetzte Poly-(2-acrylamido-2-methylpropan-sulfonsäure) in einer Konzentration vorliegt, die vorzugsweise im Bereich von 0,01 bis 10 Gew.-% und, noch bevorzugter, von 0,05 bis 5 Gew.-%, jeweils bezogen auf das Gesamtgewicht der Zusammensetzung, liegt.

11. Zusammensetzungen nach einem der Ansprüche 1 bis 10, dadurch gekennzeichnet, daß das Oxidationsmittel unter Wasserstoffperoxid, Harnstoffperoxid, den Perboraten, den Persulfaten und den Gemischen dieser Oxidationsmittel ausgewählt ist.

12. Zusammensetzungen nach einem der Ansprüche 1 bis 11, dadurch gekennzeichnet, daß das Oxidationsmittel Wasserstoffperoxid ist.

13. Zusammensetzungen nach Anspruch 12, dadurch gekennzeichnet, daß die Konzentration des Wasserstoffperoxids im Bereich von 0,5 bis 40 Volumina und vorzugsweise von 2 bis 30 Volumina liegt.

14. Zusammensetzungen nach Anspruch 11, dadurch gekennzeichnet, daß die Konzentration der Verbindung, aus der durch Hydrolyse Wasserstoffperoxid freigesetzt werden kann, im Bereich von 0,1 bis 25 Gew.-%, bezogen auf das Gesamtgewicht der oxidierenden Zusammensetzung, liegt.

15. Zusammensetzungen nach einem der Ansprüche 1 bis 14, dadurch gekennzeichnet, daß sie wässerige Zusammensetzungen sind und daß der pH-Wert der gesamten Zusammensetzung im Bereich von 1 bis 13 und insbesondere von 2 bis 12 liegt.

16. Zusammensetzungen nach einem der Ansprüche 1 bis 15, dadurch gekennzeichnet, daß sie in Form eines transparenten Gels einer Viskosität im Bereich von 50 Centipoises bis 100 Poises und vorzugsweise von 75 Centipoises bis 5 Poises vorliegen.

17. Verfahren zum oxidativen Färben von Keratinfasern, insbesondere menschlicher Keratinfasern, wie der Haare, bei dem eine Färbemittelzusammensetzung, die in einem zum Färben von Keratinfasern geeigneten Träger mindestens ein Farbstoffvorprodukt eines Oxidationsfarbstoffes und eine oxidierende Zusammensetzung wie in einem der Ansprüche 1 bis 16 definiert enthält, eingesetzt wird.

18. Verfahren zur Färbung nach Anspruch 17, bei dem die Färbemittelzusammensetzung bei der Anwendung mit der oxidierenden Zusammensetzung gemischt wird; das erhaltene Gemisch wird anschließend auf die Keratinfasern aufgebracht, und man läßt ungefähr 3 bis 50 Minuten und vorzugsweise ungefähr 5 bis 30 Minuten einwirken, spült das Gemisch dann aus, wäscht mit Haarwaschmittel, spült erneut und trocknet.

19. Verfahren zur Färbung nach Anspruch 17, bei dem die Färbemittelzusammensetzung und die oxidierende Zusammensetzung mit oder ohne zwischenzeitliches Ausspülen nacheinander aufgetragen werden.

20. Verfahren zur Behandlung von Keratinmaterialien und insbesondere der Haare, das eine dauerhafte Verformung dieser Keratinmaterialien, insbesondere in Form von dauergewellten Haaren, zum Ziel hat und das die folgenden Schritte umfaßt:

(i) Auftragen einer reduzierenden Zusammensetzung auf das zu behandelnde Keratinmaterial, das vor, während oder nach diesem Auftragen unter mechanische Spannung gesetzt wird,
(ii) gegebenenfalls Spülen des Keratinmaterials,
(iii) Auftragen einer oxidierenden Zusammensetzung wie in einem der Ansprüche 1 bis 16 definiert auf das gegebenenfalls gespülte Keratinmaterial
und
(iv) gegebenenfalls erneutes Spülen des Keratinmaterials.

21. Verfahren zur Entfärbung von Keratinmaterialien und insbesondere der Haare, das folgende Schritte umfaßt:

(i) Auftragen einer oxidierenden Zusammensetzung nach einem der Ansprüche 1 bis 16 auf das Keratinmaterial
und
(ii) Spülen des so behandelten Keratinmaterials.

22. Verwendung eines vernetzten und neutralisierten Poly-(2-acrylamido-2-methylpropan-sulfonsäure)-Polymeren wie in den Ansprüchen 1 bis 10 definiert als Verdickungsmittel und/oder als Gelbildner in einer kosmetischen und/oder dermatologischen Zusammensetzung, die mindestens ein Oxidationsmittel enthält, das unter Wasserstoffperoxid und den Verbindungen, aus denen durch Hydrolyse Wasserstoffperoxid freigesetzt werden kann, sowie den Gemischen dieser Oxidationsmittel ausgewählt ist.